# EUROPEAN PATENT SPECIFICATION

(11) **EP 3 256 079 B1**
(45) Date of publication and mention of the grant of the patent: **03.05.2023**
(21) Application number: 16708834.3
(22) Date of filing: 12.02.2016
(51) Int. Cl.: A61F 2/44

(54) **SPINAL IMPLANTS WITH ENGINEERED CELLULAR STRUCTURE AND INTERNAL IMAGING MARKERS**
WIRBELSÄULENIMPLANTATE MIT MANIPULIERTER ZELLSTRUKTUR UND INTERNEN BILDGEBUNGSMARKERN
IMPLANTS RACHIDIENS AYANT UNE STRUCTURE CELLULAIRE ARTIFICIELLE ET DES MARQUEURS D'IMAGERIE INTERNES

(30) Priority: 13.02.2015 US 201562116243 P
(43) Date of publication of application: 20.12.2017
(73) Proprietor: Eit Emerging Implant Technologies GmbH, 78573 Wurmlingen (DE)
(72) Inventor: LAMERIGTS, Nancy, 6099 EB Beegden (NL); WOLFS, Jasper, 2342 BC Oegstgeest (NL); ARTS, Mark, 2512 VA Den Haag (NL); EISEN, Guntmar, 78532 Tuttlingen (DE); HEIKENS, Martijn, 7555 RJ Hengelo (NL)
(74) Representative: Murgitroyd & Company
(86) International application number: PCT/US2016/017663
(87) International publication number: WO 2016/130878

(56) References cited:
- WO-A1-2008/131310
- WO-A1-2010/019799
- WO-A2-2008/070355
- WO-A2-2008/082766
- US-A1- 2008 206 297
- US-A1- 2011 012 280
- US-A1- 2015 018 956

## Description

### CROSS-REFERENCE TO RELATED APPLICATIONS

This application claims priority to U.S. Provisional Patent Application Serial No. 62/116,243, filed February 13, 2015.

### FIELD OF THE INVENTION

The present disclosure relates to orthopedic implants, and more particularly to spinal implants that facilitate fusion of bone, such as interbody fusion cages for fusing adjacent vertebral segments of a spine, or vertebral body replacement (VBR) implants for replacing entire vertebral bodies. Even more particularly, the present disclosure relates to such spinal fusion implants having an engineered, porous cellular structure for enhanced osteosynthesis, and an internal imaging marker for ease and precision of navigation and implantation.

### BACKGROUND OF THE INVENTION

The use of fusion-promoting interbody implantable devices, often referred to as fusion or spinal cages, is well known as the standard of care for treatment of certain spinal disorders or diseases. Depending on the particular disorder or disease, it may be necessary to stabilize a weakened or damaged spinal region by reducing or inhibiting mobility in the area to avoid further progression of the damage and/or to reduce or alleviate pain caused by the damage or injury. In other cases, it is desirable to join together the damaged vertebrae and/or induce healing of the vertebrae. Vertebral body replacement (VBR) implants are known to be used to bridge large anatomical abnormalities between non-adjacent vertebral bodies. Typically, these types of implantable spinal fusion devices comprise metal framework that provides the necessary mechanical or structural scaffold to restore and maintain normal disc height, or replace the diseased vertebral segment of the spine.

Many in-vitro and in-vivo studies on bone healing and fusion have shown that porosity is necessary to allow vascularization, and that the desired infrastructure for promoting new bone growth should have a porous interconnected pore network with surface properties that are optimized for cell attachment, migration, proliferation and differentiation. At the same time, there are many who believe the implant's ability to provide adequate structural support or mechanical integrity for new cellular activity is the main factor to achieving clinical success, while others emphasize the role of porosity as the key feature. Regardless of the relative importance of one aspect in comparison to the other, what is clear is that both structural integrity to stabilize, as well as the porous structure to support cellular growth, serve as key components to proper and sustainable bone regrowth.

Current manufacturing techniques now allow greater freedom to customize implantable devices to the user or patient's needs. Techniques such as three-dimensional (3D) printing, selective laser melting (SLM), electron beam melting (EBM), layer deposition, rapid manufacturing, and other comparable manufacturing techniques available today allow these implants to possess a variety of features not otherwise possible in years past. In some cases, the implants may be manufactured such that a unitary body can comprise a solid portion integrated with a non-solid, or porous, portion. This type of structure could mimic a natural bone structure to enhance fusion and improve clinical outcomes.

Thus, it is desirable to provide an implant that has the necessary mechanical strength or structural integrity to restore disc height or vertebral alignment to the spinal segment to be treated, as well as interconnected pores and other surface features to allow proper cellular activity and ultimately fusion of that segment. Even more desirable would be an implant as described, with internal imaging markers, to help the user navigate through visualization techniques and properly align the implant during insertion.

Although the following discussion focuses on spinal implants or prostheses, it will be appreciated that many of the principles may equally be applied to other structural body parts requiring bone repair or bone fusion within a human or animal body.

From US 2011/012280 implants for use in interbody fusion and methods of manufacturing such implants are known. In particular, said implants are formed from synthetic bone polymers. The implants comprise an interbody spacer produced by means of curation of said polymer using a molding process.

Furthermore, cellular scaffolds for providing tissue repair and regeneration, in particular to increase bone integration, are known from WO 2008/082766. Said scaffolds comprise a series of microstructures of a single type made from a specific polymeric material.

### SUMMARY

An example provides spinal fusion devices, such as interbody fusion and vertebral replacement devices. These devices are configured for insertion into a patient's intervertebral disc space for restoring disc height, or for treating a deficient vertebral column by replacing and/or restoring the bony anatomy, respectively. These fusion devices may be provided with an engineered cellular structure component, which means they can have an interconnected network of pores and other micro and/or nano sized structures that take on a mesh-like configuration or appearance. In addition, these fusion devices can also include internal imaging markers that allow the user to properly align the device and generally facilitate insertion through visualization during navigation. The imaging marker may be configured to be visualized as a solid body relative to the mesh-like structure under x-ray, fluoroscopy or CT scan, for example.

In an embodiment, a spinal fusion device is provided comprising: a main body comprising a solid framework, at least one external surface panel comprising an engineered cellular structure having a mesh-like appearance that is integrated within the solid framework, and internally located imaging marker that is attached to the solid framework by the engineered cellular structure, wherein the imaging marker is configured to appear as a solid body under visualization, in which the device is metallic or a ceramic, and is formed by an additive manufacturing technique such that the solid framework, engineered cellular structure, and imaging marker are integrally formed into a unitary body having combined solid and porous components.

The spinal fusion device may be one of a PLIF, TLIF, CIF, ALIF, LLIF or OLIF cage, or a vertebral replacement device. The spinal fusion device may be inserted into a patient's intervertebral disc space for restoring disc height to the spinal column. The imaging marker may be rod-shaped or T-shaped. The device may include a pair of imaging markers and in some embodiments, the markers may overlap. The imaging marker may be cross-shaped or H-shaped, comprise a rounded bead, a V-shaped notch, an X-Y-Z coordinate axis, or a solid column. The device may be formed of titanium, tantalum, barium, or an alloy thereof, or a printable ceramic.

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.
FIG. 1 shows a series of electron microscopic pictures with various magnifications of surface features of the implantable devices of the present disclosure, in which:
   FIG. 1A shows a network of macropores;
   FIG. 1B shows a network of microstructures including surface roughness; and
   FIG. 1C shows a network of nanostructures.
FIG. 2 shows an exemplary embodiment of an interbody fusion cage for the cervical spine, in which:
   FIG. 2A shows a perspective side view; and
   FIG. 2B shows a perspective posterior view.
FIG. 3 shows an exemplary embodiment of a cervical cage of the present disclosure, in which:
   FIG. 3A shows a perspective view of the cervical cage comprising a solid external shell component and an internal engineered cellular component with internal imaging markers; and
   FIG. 3B shows the internal engineered cellular component without the solid external shell component.
FIG. 4 shows exemplary embodiments of various internal imaging markers useful with the interbody fusion devices of the present disclosure, in which:
   FIGS. 4A to 4F illustrate various exemplary embodiments of internal imaging markers that differ in size, location and quantity for use in the devices of the present disclosure.
FIG. 5 shows an exemplary embodiment of a posterior lumbar interbody fusion device (PLIF) of the present disclosure, in which:
   FIG. 5A is a partial cutaway perspective view of the posterior lumbar interbody fusion device;
   FIG. 5B is another perspective view of the device of FIG. 5A;
   FIG. 5C is a partial cutaway side view of the device of FIG. 5A;
   FIG. 5D is a side view of the device of FIG. 5C;
   FIG. 5E is a partial cutaway top-down view of the solid model of FIG. 5A; and
   FIG. 5F is another top-down view of the solid model of FIG. 5E.
FIG. 6 shows an exemplary embodiment of a cervical interbody fusion (CIF) device having a metal framework and integrated mesh-like structure, in which:
   FIG. 6A shows a perspective view of a metal framework of an exemplary embodiment of a cervical interbody fusion (CIF) device;
   FIG. 6B is another perspective view of the metal framework of FIG. 6A;
   FIG. 6C shows a partial cutaway view of the metal framework and integrated mesh-like structure of the device of FIG. 6A;
   FIG. 6D is another perspective view of the device of FIG. 6C;
   FIG 6E shows a partial cutaway top-down view of the device of FIG. 6A;
   FIG. 6F shows another perspective view of the device of FIG. 6A.
FIG. 7 shows an exemplary embodiment of a transforaminal lumbar interbody fusion (TLIF) device; in which:
   FIG. 7A is a perspective view of an exemplary embodiment of a transforaminal lumbar interbody fusion (TLIF) device, in which:
   FIG. 7B shows a top view of the device of FIG. 7A;
   FIG. 7C shows a partial cutaway side view of the device of FIG. 7A; and
   FIG. 7D shows another partial cutaway side view from a different angle of the device of FIG. 7A.

### DESCRIPTION OF THE EMBODIMENTS

The present disclosure provides various spinal fusion devices, such as interbody fusion devices, or cages, for insertion between adjacent vertebrae, or vertebral body replacement devices for replacement of a deficient vertebral corpus or corpora of a spinal column. The devices can be configured for use in either the cervical or lumbar region of the spine. These cages and replacement devices can restore and maintain intervertebral height of the spinal segment to be treated. The devices may be manufactured using selective laser melting (SLM) techniques, a form of additive manufacturing The devices may also be manufactured by other comparable techniques, such as for example, 3D printing, electron beam melting (EBM), layer deposition, and rapid manufacturing. With these production techniques, it is possible to create an all-in-one combined solid and porous product. For example, the cages and replacement devices can be made with an engineered cellular structural component that includes a network of pores, microstructures and nanostructures to facilitate osteosynthesis.

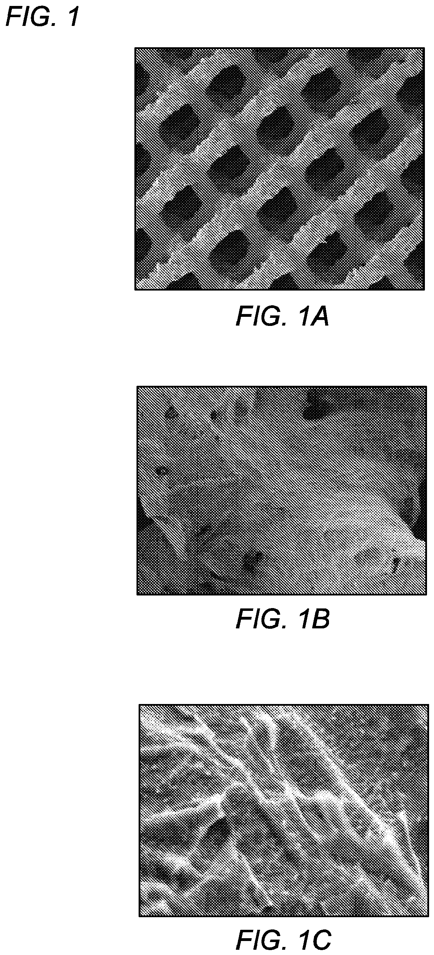

As mentioned, the devices of the present disclosure can have an engineered cellular structural component that is integrated within a solid metal framework or body. This cellular structural component can take various forms. Referring now to FIG. 1, a series of electron microscopic pictures show various features that can be a part of the cellular structural component. For example, as shown in FIG. 1A, the devices may have a network of pores, and more specifically a network of macropores. In addition, the surface may be roughened as shown in FIG. 1B to provide a microstructure that can be porous and facilitate adhesion of cells to enhance bioactivity. As also shown in FIG. 1C, the cellular structural component can include nanostructures. These nanostructures may be achieved by chemical surface treatments or with roughened surfaces, particularly metal or metal-alloy surfaces. The combination of varying sized structures and pores helps facilitate osteosynthesis. In addition, the combination of the various features creates a mesh-like network that is suitable for bone growth.

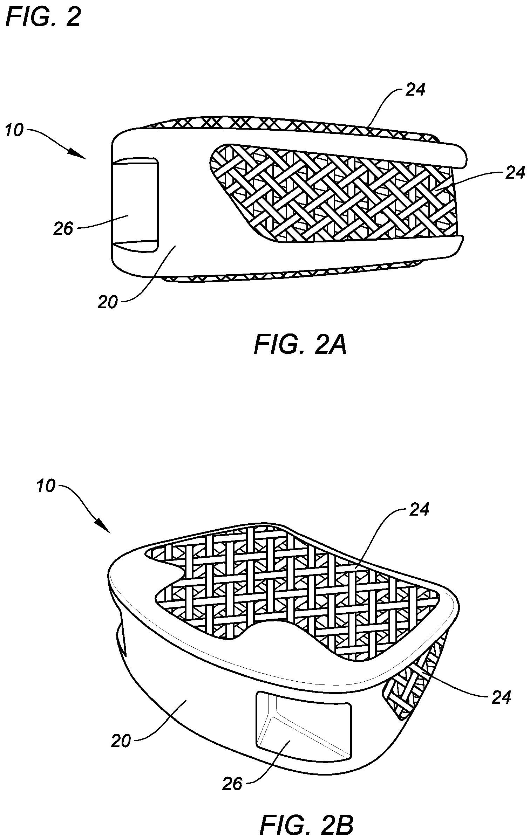

FIG. 2 shows an exemplary embodiment of an interbody fusion cage 10 configured for the cervical spine having an engineered cellular structural component, as described above. It is relevant to note in both FIGS. 2A and 2B that the engineered cellular structural component 24 is integral with the solid body framework or structure 20 of the device 10. In other words, these are unitary body devices that have been specifically created to have a roughened, porous surface on certain portions of the device to allow for bony ingrowth into the devices. As shown, the cervical fusion cage 10 comprises a solid body portion 20 and an integrated mesh-like component 24. The mesh-like component 24 is seen as a plurality of porous panels on the outer surface of the device 10, with the solid body portion 20 bordering around the panels. These panels may be configured to face external to the device 10 in order to facilitate bony ingrowth. However, the device 10 is an integral, unitary body and the mesh-like component 24 may be formed within the solid body portion 20. Slots or grooves 26 may be provided for gripping with an insertion tool or instrument.

These devices take advantage of current manufacturing techniques that allow for greater customization of the devices by creating a unitary body having both solid and porous features in one. These same manufacturing techniques may be employed to provide these devices with an internal imaging marker. A device may comprise a single marker, or a plurality of markers. These internal imaging markers greatly facilitate the ease and precision of implanting the devices, since it is possible to manufacture the device with one or more internally embedded markers for improved visualization during navigation and implantation.

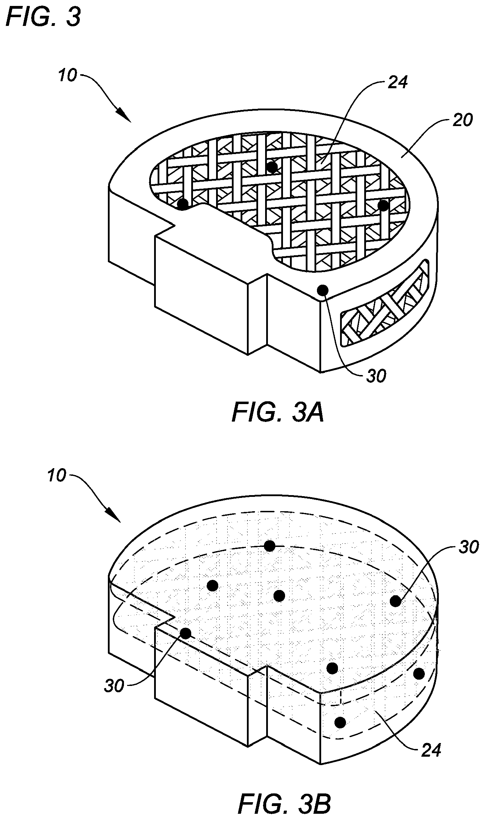

As shown in FIG. 3, the markers 30 may comprise solid body structures that are strategically located within the spinal devices, within the interior of the device, and in some cases in proximity to the mesh-like engineered cellular structural component or panel 24. The markers 30 may be visualized under X-ray, fluoroscopy, CT scan, or other known visualization techniques. In this sense, the marker 30 may not only be a solid metal body, but the 30 markers could also be a particular shape or geometry (pattern of bodies), such as a 3D shape, or a shape suitable for coordinate mapping under navigation. For instance, in FIGS. 3A and 3B, in a cutaway view of an exemplary cervical cage 10 comprising a solid external shell component 20 and an internal engineered cellular component 24 with internal imaging markers 30, the markers 30 may be rounded beads, and located around the perimeter of the device body 10, to assist the surgeon in aligning the device during the implantation process. These beads 30 would show up under visualization during navigation, such as in x-rays, for example, providing the surgeon a reference framework for locating and ultimately placing the device in situ. Suitable materials for the devices, and accordingly for the imaging markers as well since they are all an integral unit, include medical grade metals such as titanium, barium, tantalum, and other known metals and their alloys (typically in powder form), as well as ceramics (e.g., printable ceramic materials), that are able to be utilized by the production or manufacturing techniques described above such as selective laser melting or other printing processes.

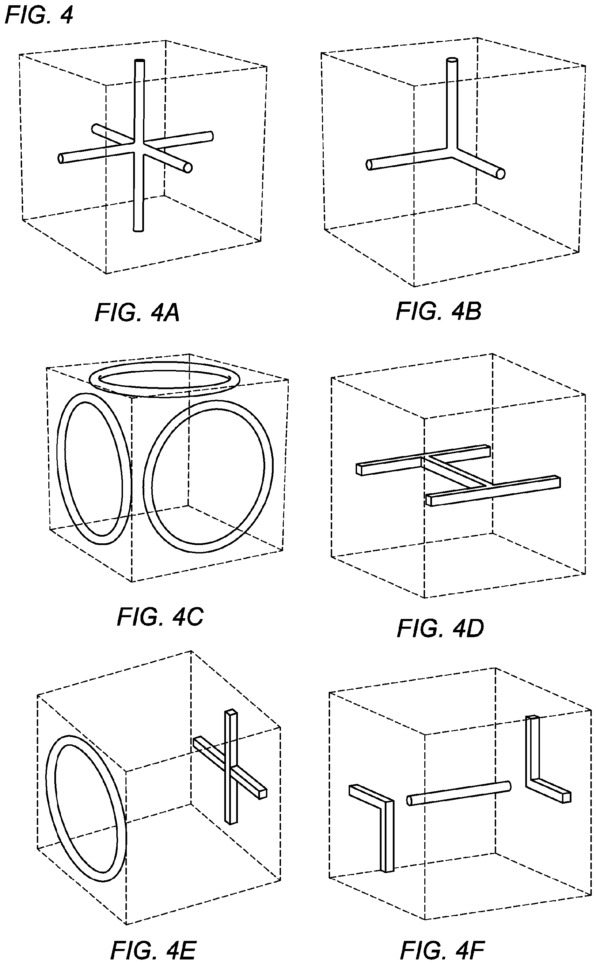

The markers may take various shapes and forms individually, and various geometries or patterns when more than one marker is provided, as shown in FIG. 4. FIG. 4A suggests that the marker may take a cruciform or cross shape. FIG. 4B suggests that the marker may be a simple X-Y-Z coordinate axis. FIG. 4C suggests that you may use two or more markers together, such as in a cluster, wherein each marker comprises a circle such that the identification of a circle on X-ray examination would suggest that the device is perpendicular. FIG. 4D suggests that the marker may be an H-frame. FIG. 4E suggests that the markers may be an "O" or "X". These types of structures can be used as orientation guides. For example, when the user sees a perfect circle under x-ray, the user can assume that the device is in a perpendicular orientation. FIG. 4F suggests that a combination of markers may cooperatively work together to produce the desired result, which is the proper orientation of the device in situ.

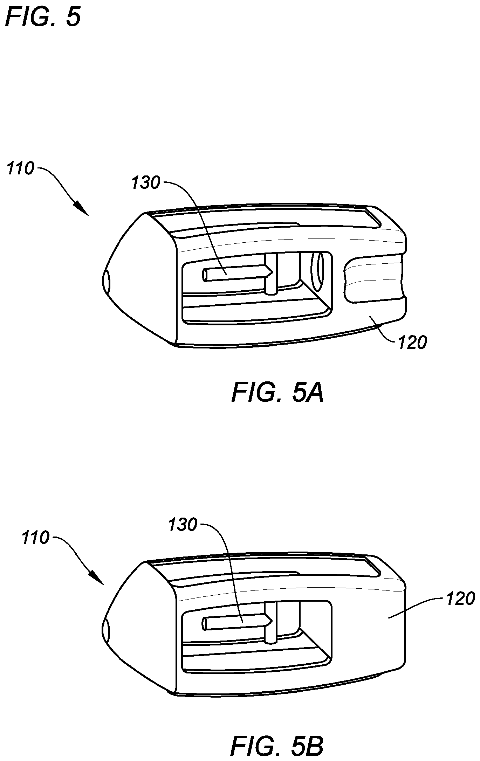

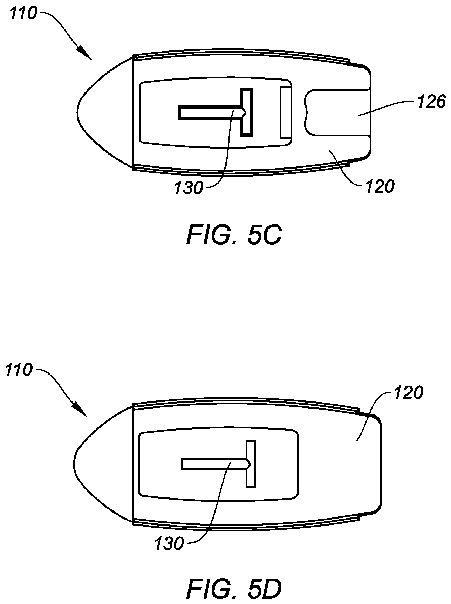

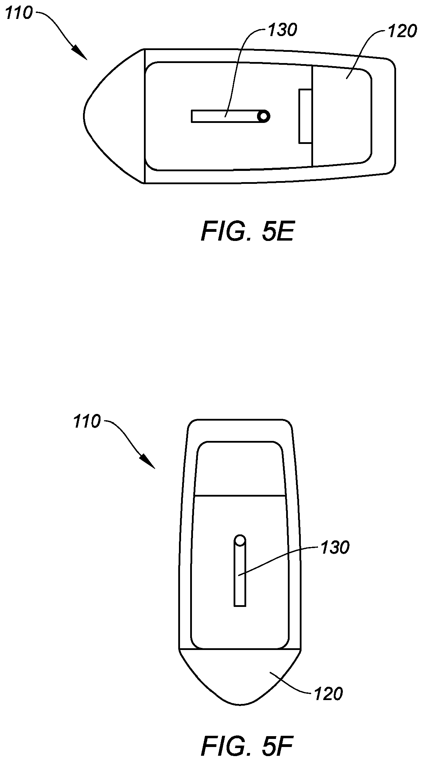

A variety of spinal implants may be provided by the present disclosure, including interbody fusion cages and/or vertebral body replacement devices for use in either the cervical or lumbar region of the spine. Although only a cervical cage 10 is illustrated with an engineered cellular structure, it is contemplated that the following interbody fusion cages shown in FIG. 5, 6 and 7 (corresponding to a posterior lumbar interbody fusion (PLIF) device, a cervical interbody fusion (CIF) device, and a transforaminal lumbar interbody fusion (TLIF) device, respectively) would also have the same type of cellular structural component. However, merely for convenience of the reader to visualize the concepts of the present disclosure, the following drawings illustrate the solid body shell of these devices for better visualization. It is to be understood that the exemplary devices shown in the drawings would have an integrated mesh-like engineered cellular structure or component in its final form, and that the cellular component is omitted for purposes of illustration only. Further, the principles of the present disclosure apply to other forms of interbody fusion cages not illustrated here, such as anterior lumbar interbody fusion (ALIF) cages, lateral lumbar interbody fusion (LLIF) cages, and oblique lumbar interbody fusion (OLIF) cages as well as in vertebral replacement devices, created with the aforementioned production methods.

FIGS. 5A to 5F illustrate various views of an exemplary posterior lumbar interbody fusion (PLIF) cage 110 of the present disclosure. In this embodiment, the marker 130 may comprise a T-bar located within the interior of the device 110. Since the drawings omit the mesh-like engineered cellular component, the marker 130 appears to be unattached to the rest of the cage body; however, as previously mentioned this is not the case in the cage's final form as the mesh-like structure will be integral with the marker 130 and the solid body frame 120 of the device 110. A slot or threaded bore 126 may be provided for attachment to an insertion tool or instrument.

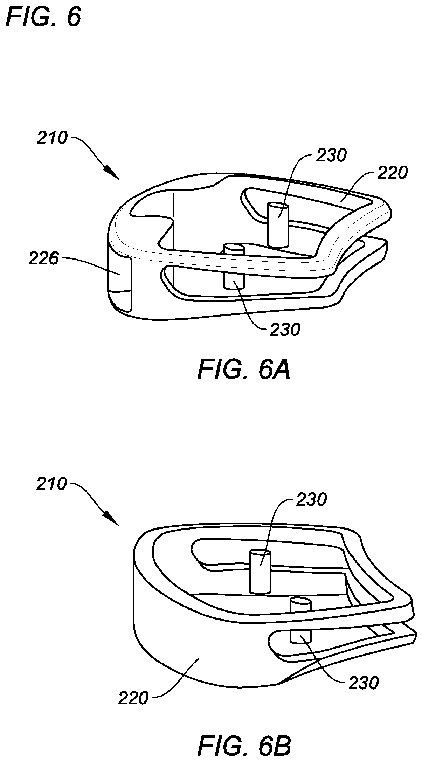

FIGS. 6A to 6F illustrate various views of an exemplary cervical interbody fusion (CIF) cage 210 of the present disclosure. The metal framework can be similar to the one shown in the devices of FIG. 2. In this embodiment, the markers 230 may comprise a pair of solid metal columns. The markers 230 are attached to the solid body frame component 220 of the cage 210 by the engineered cellular structure 224 (indicated by the broken line). Slots or grooves 226 may be provided to allow an insertion tool or instrument to grab the device for implantation.

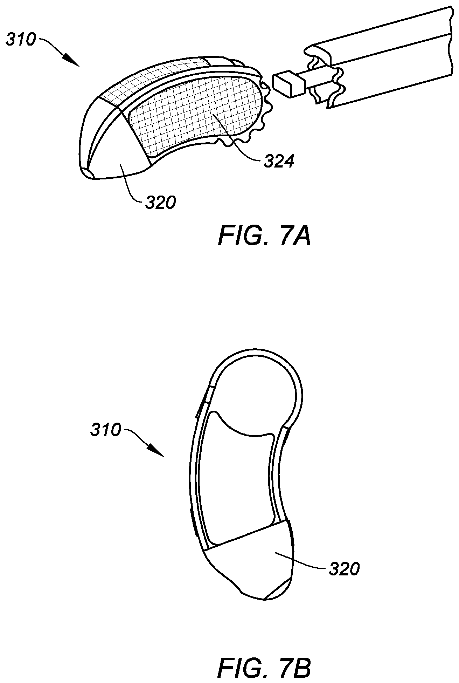

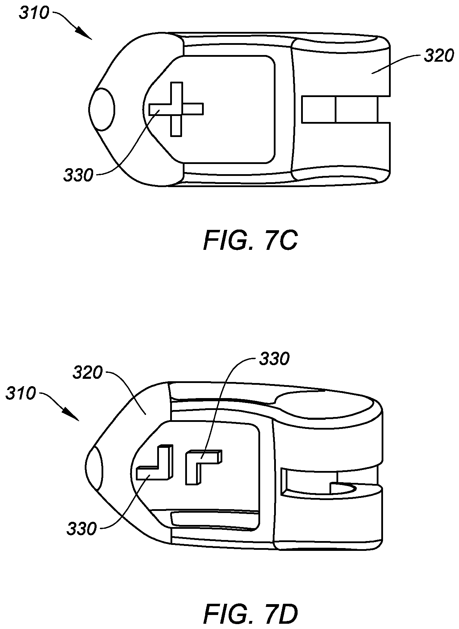

FIGS. 7A to 7D illustrate various views of an exemplary transforaminal lumbar interbody fusion (TLIF) cage 310 of the present disclosure. In this embodiment, the marker 330 may comprise a cross (FIG. 7C) or a pair of opposed V-notches (FIG. 7D). The marker(s) 330 are connected to the solid body frame component 320 of the cage 310 by the mesh-like engineered cellular component 320 shown in FIG. 7A.

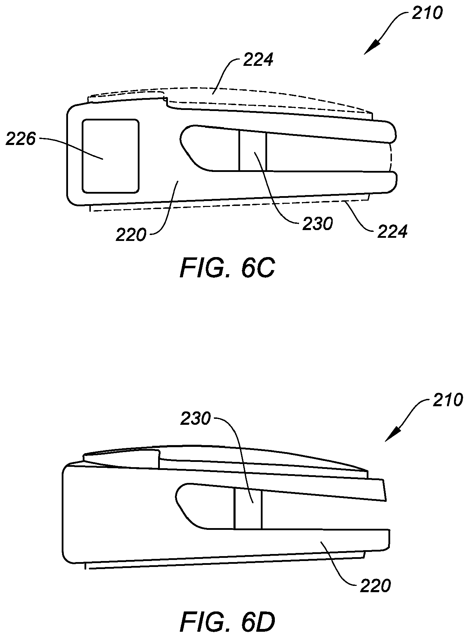

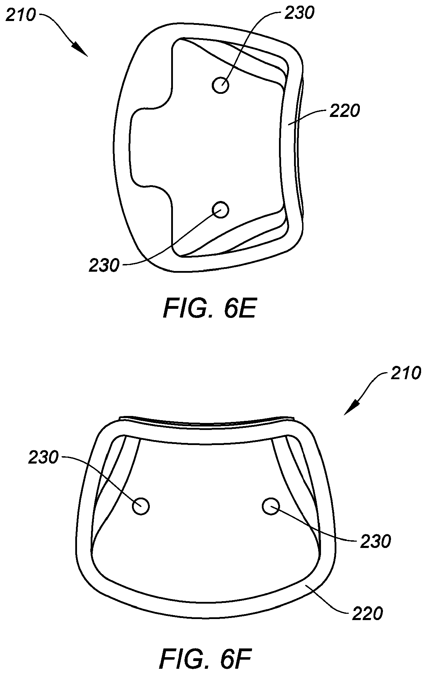

Of course, it is understood that the marker may take a different shape or form, without departing from the spirit of the invention. In addition, the solid models depicted in FIGS. 6, 7 and 8 are shown without the engineered cellular structure component in order to show the inside imaging marker; it is understood that these open spaces would otherwise be closed with the engineered cellular structural component to provide a fully closed system. Likewise, in examples not forming part of the claimed invention, there are other types of devices that could utilize these features and accordingly, the teachings in this disclosure are not to be limited to the spine. These same principles would apply equally to other fusion promoting devices for bony repair or regrowth, such as for orthopedic plates or screws, or any device which would benefit from improved visualization during navigation and implantation, and beyond such as post-operative assessments where the surgeon may want to have better visual identification of the device.

Other embodiments of the disclosure will be apparent to those skilled in the art from consideration of the specification and practice of the disclosure provided herein. It is intended that the specification and examples be considered as exemplary only.

## Claims

1. A spinal fusion device (10) comprising:
a main body (20) comprising a solid framework, at least one external surface panel comprising an engineered cellular structure (24) having a mesh-like appearance that is integrated within the solid framework, and internally located imaging marker (30) that is attached to the solid framework by the engineered cellular structure,
wherein the imaging marker is configured to appear as a solid body under visualization,
in which the device (10) is metallic or a ceramic, and is formed by an additive manufacturing technique such that the solid framework (20), engineered cellular structure (24), and imaging marker (30) are integrally formed into a unitary body having combined solid and porous components.

2. The device of claim 1, wherein the device (10) is one of a PLIF, TLlF, CIF, ALIF, LLIF or OLIF cage, or a vertebral replacement device.

3. The device of claim 1, wherein the imaging marker (30) is rod-shaped or T-shaped.

4. The device of claim 1, further comprising a pair of imaging markers (30).

5. The device of claim 4, wherein the pair of imaging markers (30) overlap.

6. The device of claim 1, wherein the imaging marker (30) is cross-shaped.

7. The device of claim 1, wherein the imaging marker (30) comprises a rounded bead.

8. The device of claim 1, wherein the imaging marker (30) comprises an H-shape.

9. The device of claim 1, wherein the imaging marker (30) comprises a V-shaped notch.

10. The device of claim 1, wherein the imaging marker (30) comprises a X-Y-Z coordinate axis.

11. The device of claim 1, wherein the imaging marker (30) comprises a solid column.

12. The device of claim 1, wherein the device (10) is formed of titanium, tantalum, barium, or an alloy thereof, or a printable ceramic.

13. The device of claim 1, wherein the visualization comprises x-ray, fluoroscopy, or CT scan.

14. The device of claim 1, wherein the additive manufacturing technique comprises 3D printing.

15. The device of claim 1, wherein the engineered cellular structure (24) comprises a nanostructure.

## Patentansprüche

1. Spondylodesevorrichtung (10), umfassend:
einen Hauptkörper (20), der ein massives Grundgerüst umfasst, mindestens ein Außenoberflächenelement, das eine konstruierte zelluläre Struktur (24) mit einem netzartigen Aussehen, die innerhalb des massiven Grundgerüsts integriert ist, umfasst, und einen intern angeordneten Bildgebungsmarker (30), der durch die konstruierte zelluläre Struktur an dem massiven Grundgerüst angebracht ist,
wobei der Bildgebungsmarker so ausgebildet ist, dass er bei einer Visualisierung als Festkörper erscheint, wobei die Vorrichtung (10) metallisch oder eine Keramik ist und derart durch eine additive Herstellungstechnik gebildet wird, dass das massive Grundgerüst (20), die konstruierte zelluläre Struktur (24) und der Bildgebungsmarker (30) integriert zu einem einstückigen Körper mit kombinierten massiven und porösen Komponenten ausgebildet sind.

2. Vorrichtung nach Anspruch 1, wobei die Vorrichtung (10) einer von einem PLIF-, TLIF-, CIF-, ALIF-, LLIF- oder OLIF-Käfig oder eine Wirbelersatzvorrichtung ist.

3. Vorrichtung nach Anspruch 1, wobei der Bildgebungsmarker (30) stabförmig oder T-förmig ist.

4. Vorrichtung nach Anspruch 1, die ferner ein Paar von Bildgebungsmarkern (30) umfasst.

5. Vorrichtung nach Anspruch 4, wobei das Paar von Bildgebungsmarkern (30) überlappt.

6. Vorrichtung nach Anspruch 1, wobei der Bildgebungsmarker (30) kreuzförmig ist.

7. Vorrichtung nach Anspruch 1, wobei der Bildgebungsmarker (30) ein abgerundetes Kügelchen umfasst.

8. Vorrichtung nach Anspruch 1, wobei der Bildgebungsmarker (30) eine H-Form umfasst.

9. Vorrichtung nach Anspruch 1, wobei der Bildgebungsmarker (30) eine V-förmige Kerbe umfasst.

10. Vorrichtung nach Anspruch 1, wobei der Bildgebungsmarker (30) eine X-Y-Z-Koordinatenachse umfasst.

11. Vorrichtung nach Anspruch 1, wobei der Bildgebungsmarker (30) eine massive Säule umfasst.

12. Vorrichtung nach Anspruch 1, wobei die Vorrichtung (10) aus Titan, Tantal, Barium oder einer Legierung davon oder einer druckfähigen Keramik ausgebildet ist.

13. Vorrichtung nach Anspruch 1, wobei die Visualisierung ein Röntgen, eine Fluoroskopie oder eine CT-Abtastung umfasst.

14. Vorrichtung nach Anspruch 1, wobei die additive Herstellungstechnik ein 3D-Drucken umfasst.

15. Vorrichtung nach Anspruch 1, wobei die konstruierte zelluläre Struktur (24) eine Nanostruktur umfasst.

## Revendications

1. Dispositif de fusion rachidienne (10) comprenant :
un corps principal (20) comprenant une ossature pleine, au moins un panneau de surface externe comprenant une structure cellulaire artificielle (24) ayant un aspect de type maille qui est intégrée au sein de l'ossature pleine, et un marqueur d'imagerie (30) situé en interne qui est attaché à la structure pleine par la structure cellulaire artificielle,
dans lequel le marqueur d'imagerie est configuré pour apparaître sous forme de corps plein à la visualisation, dans lequel le dispositif (10) est métallique ou en céramique, et est formé par une technique de fabrication additive de sorte que l'ossature pleine (20), la structure cellulaire artificielle (24) et le marqueur d'imagerie (30) soient formés d'un seul tenant en un corps monobloc ayant des composants pleins et poreux combinés.

2. Dispositif selon la revendication 1, dans lequel le dispositif (10) est l'une d'une cage PLIF, TLIF, CIF, ALIF, LLIF ou OLIF, ou d'un dispositif de remplacement vertébral.

3. Dispositif selon la revendication 1, dans lequel le marqueur d'imagerie (30) est en forme de tige ou en forme de T.

4. Dispositif selon la revendication 1, comprenant en outre une paire de marqueurs d'imagerie (30)

5. Dispositif selon la revendication 4, dans lequel les marqueurs d'imagerie (30) de la paire se chevauchent.

6. Dispositif selon la revendication 1, dans lequel le marqueur d'imagerie (30) est cruciforme.

7. Dispositif selon la revendication 1, dans lequel le marqueur d'imagerie (30) comprend une bille arrondie.

8. Dispositif selon la revendication 1, dans lequel le marqueur d'imagerie (30) comprend une forme en H.

9. Dispositif selon la revendication 1, dans lequel le marqueur d'imagerie (30) comprend une encoche en forme de V.

10. Dispositif selon la revendication 1, dans lequel le marqueur d'imagerie (30) comprend un axe de coordonnées X-Y-Z.

11. Dispositif selon la revendication 1, dans lequel le marqueur d'imagerie (30) comprend une colonne pleine.

12. Dispositif selon la revendication 1, dans lequel le dispositif (10) est formé de titane, de tantale, de baryum, ou d'un alliage de ceux-ci, ou d'une céramique imprimable.

13. Dispositif selon la revendication 1, dans lequel la visualisation comprend des rayons X, une fluoroscopie, ou un tomodensitogramme.

14. Dispositif selon la revendication 1, dans lequel la technique de fabrication additive comprend une impression 3D.

15. Dispositif selon la revendication 1, dans lequel la structure cellulaire artificielle (24) comprend une nanostructure.
